# EUROPÄISCHE PATENTANMELDUNG

(11) **EP 1 281 415 A2**
(43) Veröffentlichungstag der Anmeldung: **05.02.2003**
(21) Anmeldenummer: 02017007.2
(22) Anmeldetag: 29.07.2002
(51) Int. Cl.: A61M 21/00

(54) **Büroraum und damit kombinierbare Regenerations-Kabine**

(30) Priorität: 30.07.2001 DE 10136924; 29.11.2001 DE 10158423
(71) Anmelder: bsk Büro- und Design GmbH, 90491 Nürnberg (DE)
(72) Erfinder: Haas, Norbert, 91207 Lauf (DE)
(74) Vertreter: Götz, Georg, Dipl.-Ing.

(57) **Zusammenfassung**

Büroraum, ausgebildet in einem fest im Boden verankerten Gebäude, wobei in dem Büroraum eine in sich abgeschlossene oder abschottbare Regenerations-Kabine aufgestellt ist, die abbaubar und an einem anderen Standort wiederaufstellbar und mit wenigstens blickdichten Wandungen, eine zur maschinellen Massage ausgebildeten Ruheauflage sowie mit Mitteln zur visuellen, auditiven und/oder über den Geruchssinn erfolgenden Beeinflussung einer Benutzerperson versehen ist.

## Beschreibung

Die Erfindung betrifft einen Büroraum, der in einem fest im Boden verankerten Gebäude ausgebildet und angeordnet ist. Ferner betrifft die Erfindung eine in diesem Büroraum aufstellbare Regenerations-Kabine.

Es sind Erholungs- und Regenerations-Kabinen bekannt (DE-C-199 06 452), worin der Personenentspannung dienende Liegeflächen aufgestellt sind. Zur Erholungsförderung ist in die Liegefläche eine Massageanordnung integriert. Ferner sind Mittel zur auditiven und visuellen Beeinflussung vorgesehen. Als wesentlich ist ferner herausgestellt, dass für die Kabine eine zentrale Steuerung angeordnet ist, welche die unterschiedlichen Regenerationskomponenten "Massage", "visuelle Beeinflussung" sowie "auditive Beeinflussung" aufeinander abgestimmt koordiniert und entsprechend den Personenwünschen beeinflußt. Als konkreter Anwendungsfall wird allerdings lediglich der Bereich von Autobahn-Raststätten bzw. Kraftfahrern angesprochen.

Aus DE-U-90 06 293 ist eine kombinierte Therapie-Behandlungskabine mit Schwebeliege mit integrierter Vibrationsmassage und mit Mehrkanal-Tonanlage bekannt, die zwecks Betrieb in Mietwohnungen zerlegbar ist. Aus DE-A-198 14 499 ist eine Wellness-Oase bekannt, worin eine Ansprache aller Sinneskanäle des Menschen stattfinden soll. Dazu wird die Kombination eines Luftsprudelmassagebads mit der Freisetzung von Duftölen, Farbspielen und entspannender Musik vorgeschlagen.

Jedoch ist auch bei in Büros arbeitenden Mitarbeitern ein starkes Bedürfnis nach einer täglichen, kurzzeitigen Erholungsphase unmittelbar am Arbeitsplatz während der regelmäßigen Arbeitszeit anzuerkennen. Zur Lösung der entsprechenden Erfindungsaufgabe werden der in Patentanspruch 1 angegebene Büroraum und die damit kombinierbare Regenerations-Kabine nach Patentanspruch 2 vorgeschlagen. Weitere, vorteilhafte Ausgestaltungen der den Büroraum und die Regenerations-Kabine umfassenden, allgemeinen erfinderischen Idee ergeben sich aus den nachgeordneten, abhängigen Ansprüchen.

Indem beispielsweise über eine Rahmenkonstruktion aus Leichtmetall die erfindungsgemäße Kabine leicht aufstellbar, montierbar und schnell wieder demontierbar und an einem anderen Standort wieder schnell aufstellbar ausgebildet ist, läßt sich ein sogenanntes "Raum-in-Raum-System" schaffen, mittels welchem in Büroräumen und -gebäuden ein oder mehrere Regenerationsund Erholungsoasen für arbeitsbelastete Mitarbeiter eingerichtet werden können. Damit diese sich auch seelisch-mental während einer Arbeitspause von der Arbeitsumgebung abkoppeln können, liegt es im Rahmen der Erfindung, die Regenerations-Kabine mit blickdichten Wandungen beispielsweise aus transluzentem oder auch völlig lichtundurchlässigem Material herzustellen. Mit einem derart erfindungsgemäß ausgestatteten Büroraum werden für die Büroangestellten erholsame "Inseln" für kurze Entspannungspausen geschaffen, welche die geistige und körperliche Leistungsfähigkeit für die nachfolgende Wiederaufnahme der Arbeit deutlich erhöhen.

Um Entspannung in einer grundsätzlich angespannten Arbeitssituation realisierbar und erfahrbar zu machen, ist es wichtig, von einer ganzheitlichen Sicht des Menschen und seinen Bedürfnissen auszugehen. Nur wenn Geist und Körper gleichermaßen entspannt sind, wird der gewünschte Erholungseffekt erreicht werden. Dazu liegt es im Rahmen der Erfindung, die menschlichen Sinne der Regenerations-Kabine soweit wie möglich und auch aufeinander abgestimmt anzusprechen. Deshalb ist die Bereitstellung einer beispielsweise für Mittagsschlaf geeigneten Liege bzw. Ruheauflage ein erfindungswesentlicher Ausgangspunkt.

Nach einer Alternative im Rahmen der allgemeinen erfinderischen Idee zeichnet sich die Regenerations-Kabine durch einen Massagesessel als Kernstück aus. Maschinell betriebene bzw. automatisierte Massagesessel sind an sich auf dem Markt bekannt ( vgl. Prospekt "Cumulus Die Wohlfühlsessel", www.himolla.de). So können sie mit einer Vielzahl unterschiedlicher Massageprogramme für den Abbau körperlicher Verspannungen sorgen. Körperliches Wohlbefinden ist nämlich ein Ausgangspunkt für geistige Entspannung, sowie die Wiederherstellung und den Erhalt der Leistungsfähigkeit. Der marktbekannte Massagesessel "Cumulus-Massage" besitzt fünf Massagefunktionen (Streichen, Klopfen, Kneten, Walken, Shiatsu), welche der Erholung, Entspannung, Lockerung, Vitalisierung und Stimulation des Benutzers dienen können. Die genannten Massagefunktionen können über die Maschinensteuerung des Massagesessels auch miteinander kombiniert werden.

Zur Unterstützung und Verstärkung der Massage-Entspannungswirkung ist im Rahmen der Erfindung vorgesehen, die menschlichen Sinne mit Musik, Duft und verschieden farbigem Licht anzusprechen. Trotz unterschiedlicher Geschmacksrichtungen von Mensch zu Mensch gibt es Wirkungstendenzen, die von Menschen mehrheitlich gleich empfunden werden. Nach einer besonderen Ausbildung der Erfindung ist es deshalb zweckmäßig, für jedes Programm des Massagesessels Empfehlungen hinsichtlich der Farbe, der Musik und des dazu passenden Duftes auszusprechen. Durch das Zusammenwirken der vier Komponenten "Massage", "Musik", "Duft" und "Farblicht" kommt es zu einem Synergie-Effekt, der auch an individuelle Vorlieben der Benutzer anpassbar sein kann.

Farben sind geeignet, die menschliche Psyche und darüber auch den Gesamtorganismus zu beeinflussen. Die Wirkung läßt sich, wie an sich bekannt, an veränderter Atem- und Pulsfrequenz, sowie dem Steigen und Sinken des Blutdrucks messen. In dieser Hinsicht besteht eine Ausbildung der erfindungsgemäßen Kabine darin, dass die Mittel zur visuellen Beeinflussung eine farbiges Licht ausstrahlende Leuchte aufweisen. Diese kann in weiterer Ausgestaltung der Erfindung als spezielle Farblichtlampe ausgeführt sein, welche beispielsweise je nach Massageprogramm unterschiedliche Farbtöne ausstrahlt. Für Erholungs- und Entspannungsprogramme bieten sich grüne und blaue Farbtöne an, da diese von den meisten Menschen als beruhigend, entspannend und harmonisierend empfunden werden. Zur Vitalisierung und Stimulation kommen rote und gelbe Farbtöne oder eine Mischung daraus zum Einsatz. Positive Energien werden frei und versetzen in eine optimistische und angeregte Stimmung. Eine auf dem Markt verfügbare, geeignete Farbleuchte besteht in dem Modell "Metamorfosi" der Firma Artemide GmbH, Itterpark 5, 40724 Hilden (vgl. deren Prospekt "Artemide The Human Light 2001", Seite 165 und 171).

Zur weiteren Konkretisierung des Gedankens mit der speziellen Farblichtleuchte wird nach einer Erfindungsausbildung vorgeschlagen, die Leuchte mit einer Mehrzahl unterschiedlich farbiger Lichtquellen und/oder zugeordneter Farbfilter, insbesondere dichroitische Farbfilter, zu realisieren. Über ein beispielsweise im Leuchtensockel eingebautes Display und über eine Fernbedienung kann dann der in der erfindungsgemäßen Kabine befindliche Benutzer mit der Farblichtleuchte gleichsam einen Dialog führen. Dies wird durch eine in die Farblichtleuchte integrierte Steuerung unterstützt, über welche Farbkombinationen auswählbar, einstellbar und aufeinander abstimmbar sind. Auf der Basis der Erfindung besteht eine besonders vorteilhafte Ausführung darin, die Steuerung in der Farbleuchte so auszuwählen bzw. auszubilden, dass ganze Farblichtspiele und -sequenzen einstellbar bzw. programmierbar sind. So können zum Beispiel zeitlich versetzt zunächst überwiegend grüne und blaue Farbtöne den Betrachter zu dessen Entspannung und danach mehr rote und gelbe Farbtöne zu dessen Stimulation angeboten werden.

Als praktisch vorteilhaft hat es sich erwiesen, die Farbleuchte in einem Eckbereich der erfindungsgemäßen Kabine anzuordnen. Dann kann der Betrachter auf dem Massagesessel mit einer diagonalen Blickrichtung einen ausreichenden Abstand einhalten, um Farbkombinationen und -spiel auf sich einwirken zu lassen.

Um zu unangenehmen Geräuschen oder einem hohen Lärmpegel am Arbeitsplatz einen Gegenpol zu schaffen, wird im Rahmen der Erfindung Musik zur Unterstützung des Massageprogramms eingesetzt. Dies kann beispielsweise über an sich bekannte Tonträger-Abspielgeräte erfolgen, die zweckmäßig über eine Fernsteuerung bedienbar sind.

Es ist bekannt, dass Wirkstoffe eines Duftes über den Geruchs- und Geschmackssinn aufgenommen werden und dabei über die Lunge ins Blut gelangen und dort bis zu 90 Minuten lang nachweisbar sind. Über das Blut entfaltet sich die Wirkung aufgenommener Duftstoffe im ganzen Körper. Dieser Sachverhalt wird durch eine vorteilhafte Erfindungsausbildung ausgenutzt, wonach Mittel zur Beeinflussung der Benutzerperson über deren Geruchssinn vorgesehen sind. Zu deren Realisierung lassen sich eigenständig bzw. rein mechanisch ausgeführte Geruchsspender einsetzen. Diese können beispielsweise mit einem Haltearm und einer daran angebrachten Klemmeinrichtung ausgeführt sein, worin Papier- oder Wattestoffe gehalten sind, welche zunächst in flüssiger Form liegende Duftstoffe aufsaugen können.

Als weitere optionale Komponente kann die erfindungsgemäße Kabine mit Behältnissen für Nahrungsergänzungsmittel, beispielsweise mit einer Nahrungsergänzungsflasche ausgestattet sein, welche Stärkungsflüssigkeiten mit geeigneten Rezepturen enthält.

Platz für Erholungsmöglichkeiten und Ruheoasen ist in Büros häufig knapp bemessen. Dem kann mit dem erfindungsgemäßen "Raum-in-Raum-System" leicht begegnet werden. Die erfindungsgemäße Kabine läßt sich mit einem Grundriß von etwa 5 m² realisieren und kann so an jedem beliebigen Ort aufgestellt werden. Der Rahmen für die Kabine läßt sich mit Hilfe einer Leichtmetallkonstruktion schaffen, wobei eine leichte Aufstellbarkeit erzielt wird. Dies trägt zu einer unkomplizierten Handhabung und zur Benutzerfreundlichkeit bei.

Weitere Einzelheiten, Merkmale, Vorteile und Wirkungen auf der Basis der Erfindung ergeben sich aus der nachfolgenden Beschreibung eines bevorzugten Ausführungsbeispiels der Erfindung sowie aus den Zeichnungen. Diese zeigen in jeweils perspektivischer Darstellung:
- Figur 1: das erfindungsgemäße "Raum-in-Raum-System" mit Büroraum und darin aufgestellter Regenerations-Kabine
- Figur 2: das Innere der Regenerations-Kabine.

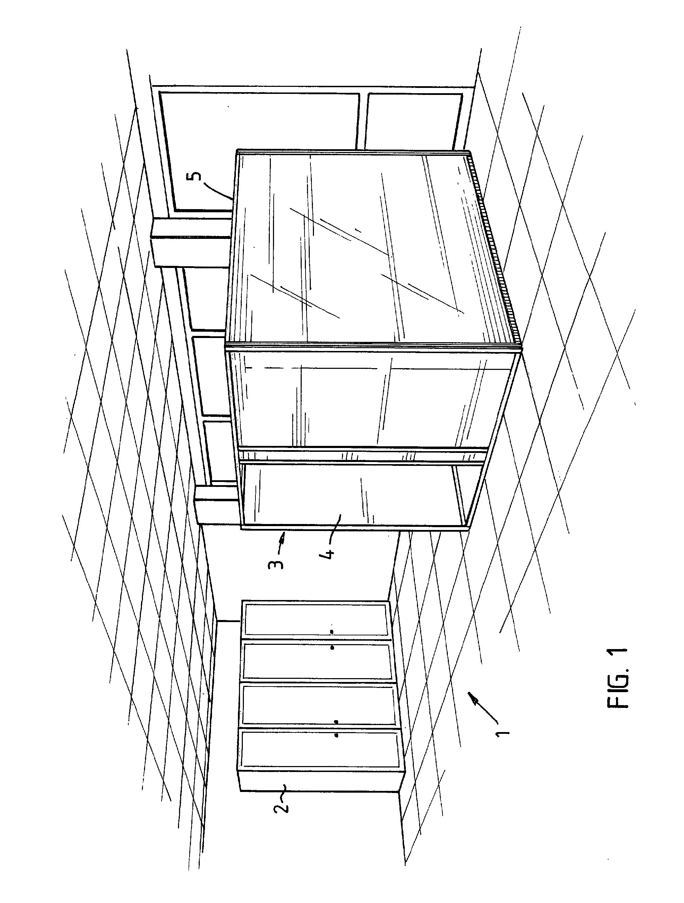

Gemäß Figur 1 befinden sich in einem geschlossenen Büroraum 1 ein Wandschrank 2 und eine erfindungsgemäße Regenerations-Kabine 3. Deren Eingang 4 kann beispielsweise mittels einer Schiebetür verschließbar sein. Vorzugsweise ist das Kabineninnere allseitig, auch von der Deckenseite 5 her, verschlossen.

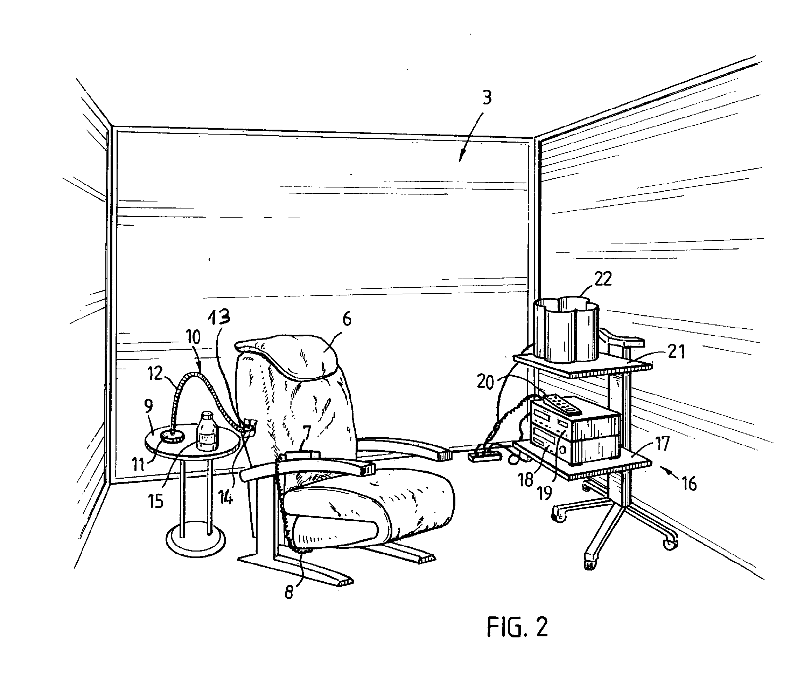

Gemäß Figur 2 bildet ein maschinell antreibbarer Massagesessel 6 das Kernstück der Inneneinrichtung der erfindungsgemäßen Kabine 3. Diese kann über ein Bedienkästchen 7, welches mit einem Kabel 8 mit einer im Massagesessel 6 integrierten Steuerung mit nachgeschaltetem Antrieb verbunden ist, in Betrieb gesetzt werden. Mit Hilfe des Bedienkästchens 7 können unterschiedliche Massageprogramme oder Kombinationen davon zum Ablauf gebracht werden. Neben dem Massagesessel 6 befindet sich ein Beistelltischchen 9, auf welchem ein Geruchsspender 10 aufgestellt ist. Dieser ist über eine flache Fußscheibe 11 auf die Tischfläche aufgesetzt. Der Geruchsspender 10 weist ferner einen dauerhaft in unterschiedliche Stellungen biegbaren Haltearm 12 auf, welcher mit einem Ende an der Fußscheibe 11 befestigt ist. Am anderen Ende ist eine (schematisch dargestellte) Klemmeinrichtung 13 ausgebildet, worin beispielsweise Papiertaschentücher 14, beträufelt mit flüssigen Duftstoffen, aufnehmbar sind. Über den flexibel biegbaren Haltearm 12 läßt sich das jeweilige Papiertaschentuch 14 von einer Benutzerperson leicht in den Bereich des Massagesessels 6 verstellen. Auf dem Beistelltischchen 9 ist ferner eine Flasche 15 mit Nahrungsergänzungsmitteln abgestellt.

An einer Innenseite der Regenerations-Kabine 3 ist ein Rollgestell 16 abgestellt, auf dessen unterem Regal 17 beispielsweise ein Kassettendeck 18, ein CD-Player 19, sowie eine Fernbedienung 20 für beide abgelegt sind. Mit letzterem läßt sich für eine im Massagesessel 6 befindliche Bedienperson bequem eine Musikauswahl treffen. Über einen (nicht gezeichneten) Kopfhörer, angeschlossen an die Musikanlage 18, 19, kann für die Bedienperson eine akustische Abschottung von der sonstigen Außenwelt realisiert werden. Auf einem oberen Regal 21 ist eine Farbleuchte 22 abgestellt, welche ebenfalls über eine Fernbedienung 20 zum Abstrahlen unterschiedlicher Farbkombinationen und - spiele angesteuert werden kann.

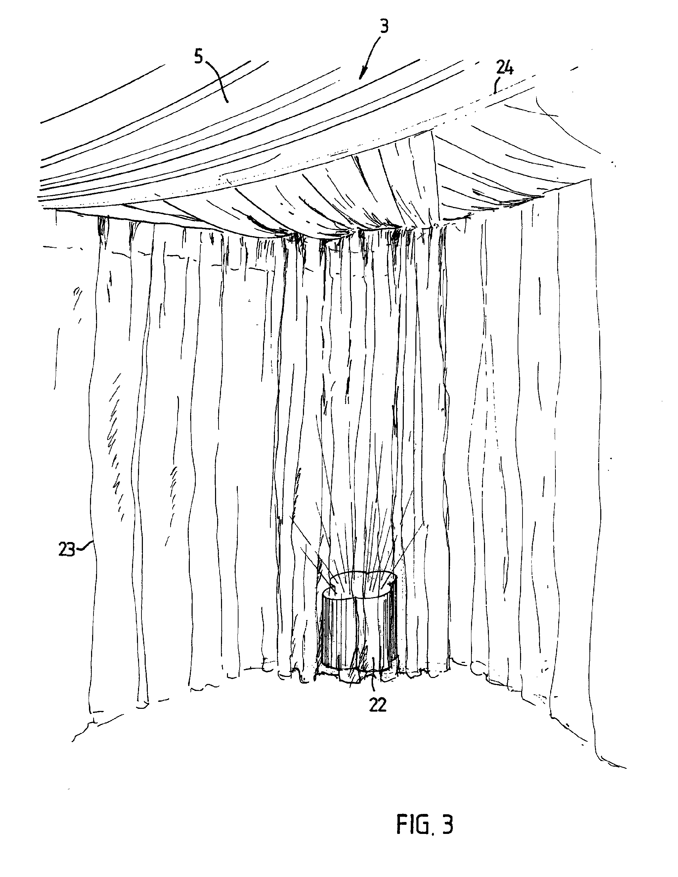

Gemäß Figur 3 sind zur Erzielung eines "weicheren" visuellen Eindrucks für eine auf dem Massagesessel liegende Person die vertikalen Wandseiten als auch die Deckenseite 5 mit Vorhängen 23 bzw. 24 abgedeckt. Dadurch wird der Einfluß von scharfkantigen Ecken auf den visuellen Gesamteindruck für die Benutzerperson gemindert. Gemäß einer besonderen Ausgestaltung kann die Leuchte 22 durch den Vorhang 23 abgedeckt sein, indem ein entsprechender Teil davon über die Leuchte 23 gezogen ist. Bei Verwendung von transluzentem Gewebe für den Vorhang 23 kann dieser noch zusätzlich Einfluß auf den Farbeindruck ausüben.

### Bezugszeichenliste

- 1: Büroraum
- 2: Wandschrank
- 3: Regenerations-Kabine
- 4: Eingang
- 5: Deckenseite
- 6: Massagesessel
- 7: Bedienkästchen
- 8: Kabel
- 9: Beistelltisch
- 10: Geruchsspender
- 11: Fußscheibe
- 12: Haltearm
- 13: Klemmeinrichtung
- 14: Papiertaschentücher
- 15: Flasche
- 16: Rollgestell
- 17: unteres Regal
- 18: Kassettendeck
- 19: CD-Player
- 20: Fernbedienung
- 21: oberes Regal
- 22: Farbleuchte
- 23: Wand-Vorhang
- 24: Decken-Vorhang

## Patentansprüche

1. Büroraum (1), ausgebildet in einem fest im Boden verankerten Gebäude, **dadurch gekennzeichnet, dass** in dem Büroraum (1) eine in sich abgeschlossene oder abschottbare Regenerations-Kabine (3) aufgestellt ist, die abbaubar und an einem anderen Standort wiederaufstellbar und mit wenigstens blickdichten Wandungen, eine zur maschinellen Massage ausgebildeten Ruheauflage sowie mit Mitteln (10;18,19;22) zur visuellen, auditiven und/oder über den Geruchssinn erfolgenden Beeinflussung einer Benutzerperson versehen ist.

2. Regenerations-Kabine (3) für den Büroraum (1) nach Anspruch 1, **dadurch gekennzeichnet, dass** die Ruheauflage als maschineller Massagesessel (6) mit Fernbedienung oder Bedientastenfeld (7) ausgeführt ist, mittels welcher beziehungsweise welchem der Massagesessel (6) zur Ausführung unterschiedlicher, in einer Steuerung gespeicherter Massageprogramme ansteuerbar ist.

3. Kabine nach Anspruch 2, **dadurch gekennzeichnet, dass** die Mittel (8,19) zur auditiven Beeinflussung Kopfhörer umfassen, welche an eine Audioquelle anschließbar sind.

4. Kabine nach Anspruch 3, **dadurch gekennzeichnet, dass** die Audioquelle als innerhalb der Kabine (3) von einer Benutzerperson manuell bedienbares, insbesondere fernsteuerbares (20) Tonträger-Abspielgerät (18,19) ausgeführt ist.

5. Kabine nach einem der vorangehenden Ansprüche, **dadurch gekennzeichnet, dass** die Mittel zur visuellen Beeinflussung eine vorzugsweise farbiges Licht ausstrahlende Leuchte (22) umfassen.

6. Kabine nach Anspruch 5, **dadurch gekennzeichnet, dass** die Leuchte (22) eine Mehrzahl unterschiedlich farbiger Lichtquellen und/oder zugeordneter Farbfilter aufweist, welche über eine manuell bedienbare Steuerung, insbesondere Fernsteuerung, auswählbar, einstellbar und/oder aufeinander abstimmbar sind.

7. Kabine nach Anspruch 6, **dadurch gekennzeichnet, dass** die Lichtquellen mit Halogenglüh- oder Leuchtstofflampen realisiert sind, denen dichroitische Farbfilter zugeordnet sind.

8. Kabine nach einem der Ansprüche 5 bis 7, **dadurch gekennzeichnet, dass** die Steuerung einen manuell ansteuerbaren Speicher für auch im zeitlichen Ablauf vorspezifierte Farbtöne und -kombinationen umfaßt.

9. Kabine nach einem der Ansprüche 5 bis 8, **dadurch gekennzeichnet, dass** die Leuchte (22) in einem Kabinen-Eckbereich angeordnet ist.

10. Kabine nach einem der vorangehenden Ansprüche, **dadurch gekennzeichnet, dass** die Mittel (10) zur über den Geruchssinn erfolgenden Beeinflussung einen Geruchsspender umfassen, der mit bezüglich Duftstoffe saugfähigen Papier- oder Wattestoffen (14) realisiert ist, welche in einer Klemmeinrichtung (14) gehalten sind.

11. Kabine nach einem der vorangehenden Ansprüche, **gekennzeichnet durch** ein oder mehrere Behältnisse (15) zur Bereithaltung von Nahrungsergänzungsmitteln.

12. Kabine nach einem der vorangehenden Ansprüche, **gekennzeichnet durch** eine Rahmenkonstruktion aus Leichtmetall.

13. Kabine nach einem der vorangehenden Ansprüche, **dadurch gekennzeichnet, dass** die Mittel zur visuellen Beeinflussung Vorhänge (23,24) oder sonstigen Wand- und/oder Deckenbehang umfassen.

14. Kabine nach Anspruch 13, **dadurch gekennzeichnet, dass** der Vorhang (23) zur Abdeckung der Leuchte (22) gegenüber der Ruheauflage angeordnet und aus transluzentem Stoff gefertigt ist.
